Europäisches Patentamt

European Patent Office

Office européen des brevets

(11) Veröffentlichungsnummer: **0 200 032 B1**

# EUROPÄISCHE PATENTSCHRIFT

(45) Veröffentlichungstag der Patentschrift: **24.07.91**

(51) Int. Cl.⁵: **C12N 9/50**, C12M 1/12

(21) Anmeldenummer: **86104712.4**

(22) Anmeldetag: **07.04.86**

(54) **Verfahren zur Abtrennung von biotechnologisch hergestellten Wertstoffen durch Querstrom-Mikrofiltration.**

(30) Priorität: **02.05.85 DE 3515650**

(43) Veröffentlichungstag der Anmeldung:
**05.11.86 Patentblatt 86/45**

(45) Bekanntmachung des Hinweises auf die
Patenterteilung:
**24.07.91 Patentblatt 91/30**

(84) Benannte Vertragsstaaten:
**AT DE GB NL**

(56) Entgegenhaltungen:
**FR-A- 2 160 853**
**FR-A- 2 201 110**

**ANALYTICA CHIMICA ACTA, Band 163, 1984, Seiten 3-12, Elsevier Science Publishers B.V., Amsterdam, NL; K.H. KRONER et al.: "On-line measurement of extracellular enzymes during fermentation by using membrane techniques"**

**CHEMICAL ENGINEERING PROGRESS, Band 80, Nr. 1, Januar 1984, Seiten 68-75, New York, US; T.J. O'SULLIVAN et al.: "Applications of ultrafiltration in biotechnology"**

(73) Patentinhaber: **Henkel Kommanditgesellschaft auf Aktien**
**Postfach 1100 Henkelstrasse 67**
**W-4000 Düsseldorf-Holthausen(DE)**

Patentinhaber: **BIOCHEMIE Gesellschaft m.b.H.**

**A-6250 Kundl Tirol(AT)**

(72) Erfinder: **Rähse, Wilfried, Dr.**
**Kolhagenstrasse 44**
**W-4000 Düsseldorf 13(DE)**
Erfinder: **Carduck, Franz-Josef, Dr.**
**Landstrasse 189**
**W-5657 Haan(DE)**
Erfinder: **Kühne, Norbert**
**Dürerstrasse 63**
**W-5657 Haan(DE)**

(74) Vertreter: **Patentanwälte Meinke und Dabringhaus Dipl.-Ing. J. Meinke Dipl.-Ing. W. Dabringhaus**
**Westenhellweg 67**
**W-4600 Dortmund 1(DE)**

EP 0 200 032 B1

CHEM. -ING. - TECHN., Band 57, Nr. 9, 1985,
Seiten 747-753, VCH Verlagsgesellschaft
mbH, Weinheim, DE; W. RÄHSE et al.:
"Mikrofiltration von Fermenterbrühen"

FOOD TECHNOLOGY, Band 38, Nr. 12, Dezember 1984, Seiten 77-111, Chicago, Illinois,
US; D.J. PAULSON et al.: "Crossflow membrane technology and its applications"

2

## Beschreibung

Die Erfindung betrifft ein Verfahren zur Abtrennung von biotechnologisch hergestellten Wertstoffen aus einer Zellsuspension, durch Querstrom-Mikrofiltration unter Verwendung poröser polymerer Rohre mit Mikroporen > 0,1 µm als Membrane, durch die die Fermenterlösung mit einer Geschwindigkeit > 2 m/s strömt und wobei ein Druckunterschied zwischen Konzentrat- und Permeatseite von weniger als 5 bar herrscht.

Eine neuere Entwicklung auf dem Gebiet der Fest/Flüssig-Trennung stellt die Querstrom-Mikrofiltration dar. ("Development studies of Crossflow microfiltration", R. Bertera, H. Steven, M. Matcalfe, The Chemical Engineer, Juni 1984, Seite 10 ff.)

Ein mögliches Einsatzgebiet dieses Membranmikrofiltrationsverfahrens liegt in der Abtrennung extrazellulärer Wertstoffe, insbesondere höheren Molekulargewichtes, von Zellkörpern sowie anderen Feststoffen.

Diese Abtrennung in Kultur- bzw. Nährlösungen, wie z. B. Fermenterlösungen, gelöster extrazellulärer Wertstoffe von Zellen, Zellbruchstücken und anderen, aus dem Nährmedium stammenden Feststoffen, ist wegen der Mikrogröße der zurückzuhaltenden Feststoffe und Bakterienzellen mit einer Größe von 0,2 bis 5 µm, der geringen Dichtedifferenz zwischen der Fermenterlösung und den abzutrennenden Feststoffen und Bakterienzellen, der Kompressibilität der Feststoffe und Bakterienzellen, sowie dem häufig nicht Newton'schen Fließverhalten der relativ hohe Viskositäten aufweisenden Fermenterlösungen äußerst problematisch.

In der großtechnischen Praxis werden daher weiterhin klassische Verfahren und Vorrichtungen zur Fest/Flüssig-Trennung, wie Filtration und Zentrifugation mit z. B. hochleistungsfähigen Seperatoren oder Filtrationsapparaten eingesetzt, obwohl diese an der Grenze der Wirtschaftlichkeit liegen und mit ihnen in diesem Bereich der Biotechnologie nur geringe Leistungen zu erreichen sind.

Von der zur Abtrennung von gelösten Stoffen mit höheren Molekulargewichten geeigneten Querstrom-Mikrofiltration sind derzeit nur wenige über den Labormaßstab hinausgehende technische Anwendungsfälle, z. B. die Eindickung von Suspensionen oder die Trennung von Emulsionen bekannt.

Der großtechnischen Anwendung der Querstrom-Mikrofiltration im Einsatzgebiet der Abtrennung bzw. Isolierung von extrazellulären Wertstoffen mit relativ hohen Molekulargewichten aus Kultur- bzw. Fermenterlösungen, wie beispielsweise der Isolierung alkalischer Protease zur Gewinnung von Enzymen, stellen sich die weiter oben bereits beschriebenen Schwierigkeiten entgegen. Bei der großtechnischen Realisation des Querstrom-Mikrofiltrationsverfahrens in diesem biotechnologischen Einsatzgebiet ergibt sich jedoch als Hauptproblem die sich auf der Membranoberfläche bildende Deckschicht, die aufgrund der Konzentrationspolarisation den Durchtritt der Wertstoffe durch die Membrane behindert und somit die Retention (Rückhaltung der Wertstoffe) verursacht sowie den Permeatfluß limitiert. Insbesondere bei Flüssigkeiten mit einem relativ hohen Feststoffanteil bildet sich diese Deckschicht bzw. verstopfen die Membranporen sehr schnell und erreicht die Retention schon nach kurzer Zeit Werte von ca. 100 %, so daß die Membrane für die Wertstoffe undurchlässig wird. Um die Poren der Membrane für die Wertstoffe wieder durchgängig zu machen, muß das an und für sich kontinuierliche Verfahren unterbrochen und ein Reinigungszyklus durchgeführt werden.

Aufgabe der Erfindung ist daher die Erzielung eines hohen spezifischen Permeatflusses bei gleichzeitig möglichst langer Verweilung der Retention nahe 0 % zur Ermöglichung der großtechnischen Querstrom-Mikrofiltration in der Biotechnologie bei der Abtrennung von biotechnologisch hergestellten extrazellulären Wertstoffen aus einer Zellsuspension, insbesondere bei der Abtrennung alkalischer Protease zur Gewinnung von Enzym, unter wirtschaftlich befriedigenden Bedingungen.

Diese Aufgabe wird dadurch gelöst, daß unter Verwendung von Mikroporendurchmesser zwischen 0,3 und 0,5 µm aufweisenden Polysulfonrohren bei einer Strömungsgeschwindigkeit der Fermenterlösung von 3 - 6 m/s parallel zur Membranoberfläche und einem Druckunterschied zwischen Konzentrat- und Permeatseite von 2 bar alkalische Protease höheren Molekulargewichts, insbesondere Enzym > 20.000 Dalton, aus einer Fermenterlösung abgetrennt wird, wobei das Verhältnis vom mittleren Porendurchmesser der Membrane zur Größe der im Konzentrat verbleibenden Mikroorganismen zwischen 0,15 und 0,85 liegt.

Das erfindungsgemäße Verfahren ermöglicht den großtechnischen Einsatz der Querstrom-Mikrofiltration, d. h. den Einsatz der Querstrom-Mikrofiltration in biotechnologischen Abtrennanlagen, die eine Filter- bzw. Membranfläche von in der Regel mehr als 10 qm aufweisen, unter wirtschaftlich akzeptablen Bedingungen. Insbesondere werden der spezifische Permeatfluß und das Retentionsverhalten bei der Anwendung der Querstrom-Mikrofiltration in dem oben beschriebenen biotechnologischen Einsatzgebiet deutlich verbessert. Dies wird insbesondere durch die gezielte Auswahl des Werkstoffes Polysulfon, der Mikroporen mit mittleren Porendurchmessern zwischen 0,3 und 0,5 µm, der Strömungsgeschwindigkeit von 3 bis 6 m/s sowie des Druckunterschiedes von 2 bar erreicht.

Polysulfonmembranen sind aufgrund der mit Ihnen zu erzielenden Werte für den Permeatfluß und die Retention für den Einsatz unter den technischen Bedingungen der Querstrom-Mikrofiltration bestens geeignet. Während z. B. Teflonmembranen, Polypropylen-Schlauchmembranen oder Ultrafiltrationsmembranen unter den technischen Bedingungen der Querstrom-Mikrofiltration nur ungenügende Permeatflüsse zulassen und schon nach kurzer Verfahrensdauer Retentionswerte von über 50 % aufweisen, sind bei den entsprechenden Polysulfonmembranen Retentionswerte nahe 0 % über eine deutlich längere Verfahrensdauer feststellbar.

Von entscheidender Bedeutung zur Erzielung eines optimalen Permeatflusses und einer geringen Retention ist die Porengrößenverteilung in den Polysulfonmembranen, charakterisiert durch den mittleren Porendurchmesser. Für die großtechnische Anwendung der Querstrom-Mikrofiltration in dem beschriebenen Einsatzgebiet ist es wesentlich, daß die Mikroporen mittlere Porendurchmesser zwischen 0,3 und 0,5 $\mu$m aufweisen, wobei das Verhältnis der mittleren Porendurchmesser der Membranen zur Größe der im Konzentrat verbleibenden Mikroorganismen zwischen 0,15 und 0,85 liegt. Gerade diese ausgewählten mittleren Porendurchmesser zwischen 0,3 und 0,5 $\mu$m gewährleisten, daß die Retention über eine Verfahrensdauer von bis zu 24 Stunden Werte nahe 0 % aufweist. Bei kleineren Porendurchmessern hat sich demgegenüber gezeigt, daß die Retention aufgrund der sich aufbauenden Deckschicht bereits nach 3 Stunden Werte von über 40 % aufweist. Bei der Auswahl von größeren Mikroporen kommt es bereits nach kurzer Verfahrensdauer zur Verstopfung der Poren durch darin hängenbleibende Bakterienzellen.

Aufgrund der für eine wirtschaftliche Durchführung der Querstrom-Mikrofiltration unbefriedigend hohen Retentionswerte von über 40 % ist bei der Querstrom-Mikrofiltration üblicherweise schon nach rund 3 Stunden eine Unterbrechung des an und für sich kontinuierlichen Verfahrens zur Durchführung eines Reinigungszyklusses notwendig. Bei dem erfindungsgemäßen Verfahren treten solch hohe Retentionswerte aber erst nach 24 Stunden auf, so daß erst dann ein Reinigungszyklus durchzuführen ist. Dies führt zu einer deutlich verbesserten Wirtschaftlichkeit des Verfahrens.

Des weiteren ist die Strömungsgeschwindigkeit in den rohrförmigen Polysulfon-Trennmembranen von wesentlichem Einfluß. Niedrige Retentionswerte können bei den eingesetzten Fermenterlösungen nur bei relativ hoher Strömungsgeschwindigkeit und wirtschaftlich nur im Bereich von 3 bis 6 m/s erzielt werden. Die Hauptschwierigkeit stellt dabei die sich auf der Membranoberfläche bildende Deckschicht dar, deren Entstehung nur durch turbulente Strömungsverhältnisse vermieden werden kann. Turbulente Strömungsverhältnisse lassen sich in den rohrförmigen Membranen bei den in einer Fermenterlösung herrschenden Viskositätsverhältnissen nur durch hohe Strömungsgeschwindigkeiten erreichen, die einerseits die Reynold'sche Kennzahl vergrößern, und andererseits durch Erhöhung der Scherkräfte die wirksame Viskosität der nicht Newton'schen Flüssigkeit so gering wie möglich halten. Diese Strömungsverhältnisse werden in der Regel nur zu Beginn der Querstrom-Mikrofiltration sichergestellt, da es im Laufe der Verfahrensdurchführung insbesondere durch teilweise rückgeführte Fermenter- bzw. Konzentratlösung zu einer Aufkonzentrierung und damit verbundenem Viskositätsanstieg kommt. Die Strömungsgeschwindigkeit kann in der großtechnischen Praxis aus wirtschaftlichen und verfahrenstechnischen Gesichtspunkten heraus aber nicht beliebig erhöht werden, weil zum einen die benötigte Pumpenenergie mit der dritten Potenz der Strömungsgeschwindigkeit ansteigt und zum anderen die bei dem Pumpvorgang in Form von Wärme eingebrachte Energie wieder über Wärmeaustauscher abgeführt werden muß. Der für eine großtechnische Anwendung optimale Bereich der Strömungsgeschwindigkeit in rohrförmigen Trennmembranen bei der Abtrennung von extrazellulären Wertstoffen im gattungsgemäßen Einsatzgebiet der Mikrofilration liegt demnach bei 3 bis 6 m/s.

Wirtschaftlich sinnvoll ist die erfindungsgemäße Verfahrensdurchführung darüberhinaus bei einem mittleren Druckgefälle zwischen Konzentrat- und Permeatseite von 2 bar. Wird das Druckgefälle auf mehr als 2 bar erhöht, erleidet die Polysulfonmembrane eine irreversible Membrankompaktion. Wird das Druckgefälle dagegen unter 2 bar abgesenkt, wird eine unnötige Absenkung des Permeatflusses in Kauf genommen.

In vorteilhafter Weise läßt sich der spezifische Permeatfluß durch einen Zusatz von 0,05 bis 1 Gew.-% an Feststoffen der Teilchengröße $\leq$ 500 $\mu$m verbessern. So läßt sich der Permeatfluß durch Zusatz von z. B. gemahlener Cellulose insbesondere fasriger Cellulose mit Teilchengrößen $\leq$ 500 $\mu$m in einer Menge von 0,05 bis 1 Gew.-% um das Doppelte bis Dreifache erhöhen. Diese Zunahme des Permeatflusses macht sich vor allem nach einer gewissen Zeit der Verfahrensdurchführung, d. h. im Bereich höherer Aufkonzentrierungen bemerkbar, obwohl die Viskosität der Fermenterlösung durch den Cellulose-Zusatz noch ansteigt. Die positive Wirkung des zugesetzten Feststoffes zeigt sich vor allem, wenn turbulente Strömungsverhältnisse nicht mehr aufrechterhalten werden können. Als geeignetster Zusatzstoff hat sich Cellulose erwiesen. Andere Feststoffzusätze, wie z. B. Natriumaluminiumsilikat oder Eisenhydroxyd führen bei den in Frage kommenden Fermenterlösungen zu keiner Steigerung des Permeatflusses.

Zweckmäßig ist es darüber hinaus, die Fermenterlösung zur Verbesserung des Permeatflusses sowie des Retentionsverhaltens mit einem Zusatz von Tensiden zu versehen.

In weiterer Ausgestaltung sieht das erfindungsgemäße Verfahren vor, daß der Querstrom-Mikrofiltration eine Ultrafiltration zur Wertstoffanreicherung nachgeschaltet wird. In einer nachgeschalteten Ultrafiltrationsanlage kann der im Permeat gelöste Wertstoff anschließend in vorteilhafter Weise angereichert bzw. aufkonzentriert werden, indem in der Ultrafiltrationsanlage unter Verwendung üblicher Membranen der Wertstoff zurückgehalten und die restlichen Bestandteile der Permeatlösung, wie z.B. Wasser, Salze sowie kleinere Moleküle durchgelassen werden. Die Querstrom-Mikrofiltration und die Ultrafiltration bilden dann ein wirtschaflich kostengünstig zusammenwirkendes Verfahren.

Zur Erzielung hoher Retentionswerte ist es günstig, daß bei der Querstrom-Mikrofiltration der alkalischen Protease ein pH-Wert zwischen 6,2 und 7,2 in der Fermenterlösung eingestellt wird. Höhere pH-Werte wären bezüglich der Enzymaktivität und der Viskosität alkalischer Protease zwar günstiger, verbieten sich aber, da bei pH-Werten oberhalb von 7,2 die Retention rapide auf unbefriedigend hohe Werte ansteigt.

Schließlich zeichnet sich das erfindungsgemäße Verfahren dadurch aus, daß die Querstrom-Mikrofiltration großtechnisch wirtschaftlich bei der Enzymabtrennung von Bakterienkulturen erfolgt, die sowohl in Gegenwart von Azid, insbesondere Natriumazid, als auch in Gegenwart von Natriumchlorid noch bei Temperaturen von 55°C zum Wachstum befähigt sind.

Ein Ausführungsbeispiel der Erfindung ist nachstehend anhand der Zeichnung beispielsweise näher erläutert. Diese zeigt in

Fig. 1    das Prinzip der Querstrom-Mikrofiltration für die Abtrennung bzw. Isolierung von extrazellulären Inhaltsstoffen aus Kultur- bzw. Fermenterlösungen und in

Fig. 2    ein vereinfachtes Verfahrensfließbild einer Querstrom-Mikrofiltrationsanlage.

Bei dem dargestellten Verfahrensprinzip zur Abtrennung von biotechnologisch hergestellten extrazellulären Wertstoffen aus einer Zellsuspension durch Querstrom-Mikrofiltration findet das rohrförmige Trennmodul 1 Verwendung. Das rohrförmige Trennmodul 1 besteht aus einem Stützrohr 2, in das ein aus Polyestergewebe bestehendes Trägerrohr 3 eingelassen ist, auf dessen Mantelinnenfläche eine trennaktive ca. 50 bis 100 µm dicke Membranschicht 4 aus Polysulfon aufgebracht ist. Das rohrförmige Trennmodul 1 weist Durchmesser zwischen 5 und 15 mm auf.

Die Kultur- bzw. Fermenterlösung 5 wird in Pfeilrichtung mit Strömungsgeschwindigkeiten von 3 bis 6 m/s über die trennaktive Membranschicht 4 geführt, wobei die Wertstoffe die trennaktive Membranschicht 4 in radialer Richtung durchdringen, während Zellen, Zellbruchstücke und Feststoffe von der trennaktiven Membranschicht 4 zurückgehalten werden. Es bildet sich ein flüssiges Konzentrat 6, welches die Zellen, Zellbruchstücke und Feststoffe enthält sowie ein flüssiges Permeat 7, worin sich der abgetrennte Wertstoff befindet. Als treibende Kraft für den Durchtritt von Flüssigkeit der Fermenterlösung 5 und der darin gelösten Wertstoffe durch die trennaktive Membranschicht 4 wirkt ein Druckgefälle. Das mittlere Druckgefälle zwischen der Konzentratseite und der Permeatseite beträgt im Mittel 2 bar. Auf der Permeatseite herrschen der tagesspezifische Luftdruck und die geodätische Höhe des offenen Ablaufs. Die trennaktive Membranschicht 4 aus Polysulfon weist Mikroporen mit mittleren Porendurchmessern zwischen 0,3 und 0,5 µm auf. Das Verhältnis dieser mittleren Porendurchmesser der Membrane 4 zur Größe der im Konzentrat 6 verbleibenden und vorher mit der Kultur- bzw. Fermenterlösung 5 herangeführten Mikroorganismen, insbesondere Bakterienkulturen, die sowohl in Gegenwart von Natriumazid als auch in Gegenwart von Natriumchlorid noch bei Temperaturen um 55°C zum Wachstum befähigt sind, liegt zwischen 0,15 und 0,85.

Bei dem abgetrennten Permeat handelt es sich insbesonders uni eine alkalische Protease, bei der ein Enzym mit einem Molekulargewicht größer 20.000 Dalton den zu gewinnenden Wertstoff darstellt.

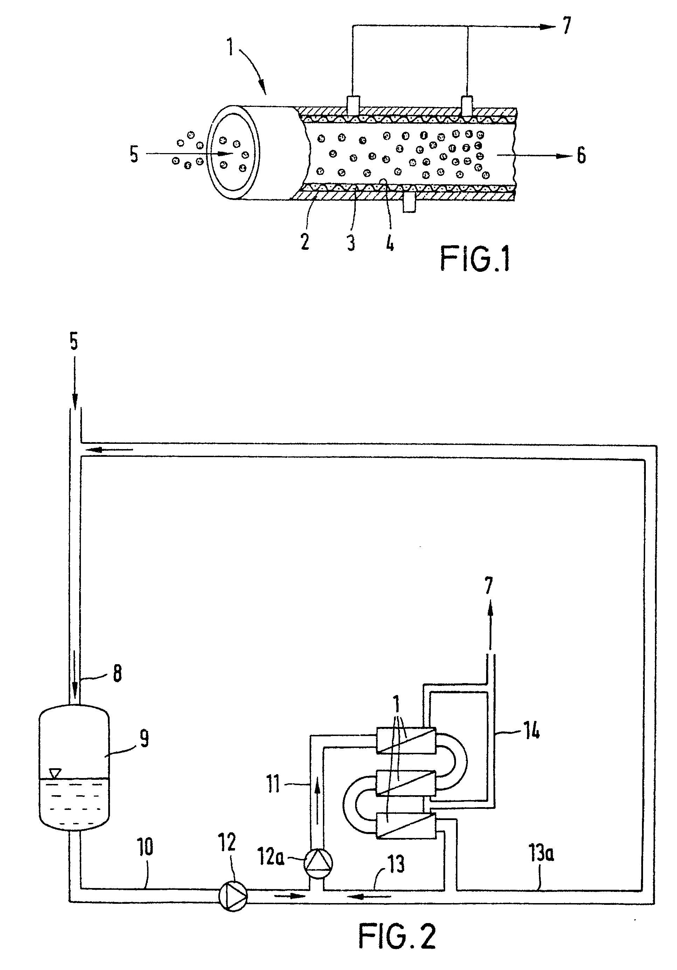

Dag rohrförmige Trennmodul 1 stellt das Herzstück des in Figur 2 dargestellten vereinfachten Verfahrensfließbildes einer Querstrom-Mikrofiltrationsanlage dar. Hierbei wird die Fermenterlösung 5 über eine Rohrleitung 8 in einen beheizbaren Vorlagebehälter 9 gefüllt, aus dem sie über Rohrleitungen 10, 11 mit Hilfe einer Pumpe 12 in der Leitung 10 und einer Pumpe 12a in der Leitung 11 in die rohrförmigen Trennmodule 1 gelangt. Drei hintereinandergeschaltete rohrförmige Trennmodule 1 bilden eine Trennstufe. In jedem der drei rohrförmigen Trennmodule 1 wird die Kultur- bzw. Fermenterlösung 5 in das Konzentrat 6 und das Permeat 7 getrennt, wobei das Konzentrat über Rohrleitungen in das jeweils nächste Modul 1 gelangt bzw. aus dem letzten Modul über die Rohrleitung 13 größtenteils in die Zulaufleitung 11 rückgeführt wird, während das Permeat aus jedem Trennmodul 1 in die Abflußleitung 14 abfließt. Ein kleinerer Teil des Konzentrats 6 gelangt über eine Abzweigleitung 13a und die Leitung 8 in den Behälter 9 zurück, wozu die Pumpleistung der Pumpe 12a entsprechend höher als die der Pumpe 12 eingestellt wird.

In weiteren Ausführungsformen kann es vorgesehen sein, das Permeat 7 über die Abflußleitung 14 einer Aufbereitungsanlage, wie z.B. einer Ultrafiltrationsanlage zur Anreicherung der Wertstoffe zuzuführen.

Auch kann es vorgesehen sein, Permeat über eine Abzweigung von der Abflußleitung 14, ggf. unter Zwischenschaltung eines Sammelbehälters, zur Durchführung von Reinigungszyklen den Leitungen 10 oder 11 zuzuführen, wobei dann der Zulauf von Fermenterlösung aus dem Vorlagebehälter 9 unterbunden wird.

Natürlich ist es auch möglich, das Querstrom-Mikrofiltrationsverfahren mit mehr als einer aus Trennmodulen 1 bestehenden Trennstufe sowie mit mehr als drei hintereinandergeschalteten Trennmodulen 1, auszuführen. Auch ist das Verfahren nicht auf die Verwendung eines Vorlagebehälters 9 beschränkt.

Insbesondere ist es ferner möglich, das Querstrom-Mikrofiltrationsverfahren mit einer batch-Diafiltration zur gezielten Flüssigkeitszugabe zu versehen, um das Konzentrat 6 z. B. mit Wasser extrahieren und den ins Permeat gelangten Flüssigkeitsanteil ausgleichen zu können. Darüber hinaus ist die Ausbildung der rohrförmigen Trennmodule 1 nicht auf den angegebenen Durchmesserbereich von 5 - 15 mm beschränkt.

## Patentansprüche

1. Verfahren zur Abtrennung von biotechnologisch hergestellten Wertstoffen aus einer Zellsuspension durch Querstrom-Mikrofiltration unter Verwendung poröser polymerer Rohre mit Mikroporen > 0,1 μm als Membrane, durch die die Fermenterlösung mit einer Geschwindigkeit > 2 m/s strömt und wobei ein Druckunterschied zwischen Konzentrat- und Permeatseite von weniger als 5 bar herrscht, dadurch gekennzeichnet, daß unter Verwendung von Mikroporendurchmesser zwischen 0,3 und 0,5 μm aufweisenden Polysulfonrohren bei einer Strömungsgeschwindigkeit der Fermenterlösung von 3 - 6 m/s parallel zur Membranoberfläche und einem Druckunterschied zwischen Konzentrat- und Permeatseite von 2 bar alkalische Protease höheren Molekulargewichts, insbesondere Enzym > 20.000 Dalton, aus einer Fermenterlösung abgetrennt wird, wobei das Verhältnis vom mittleren Porendurchmesser der Membrane zur Größe der im Konzentrat verbleibenden Mikroorganismen zwischen 0,15 und 0,85 liegt.

2. Verfahren nach Anspruch 1, gekennzeichnet durch einen Zusatz von 0,05 bis 1 Gew.-% an Feststoffen der Teilchengröße ≦ 500 μm.

3. Verfahren nach Anspruch 1 oder 2, gekennzeichnet durch einen Zusatz von Tensiden.

4. Verfahren nach einem der vorangehenden Ansprüche, dadurch gekennzeichnet, daß der Querstrom-Mikrofiltration eine Ultrafiltration zur Wertstoffanreicherung nachgeschaltet wird.

5. Verfahren nach einem der vorangehenden Ansprüche, dadurch gekennzeichnet, daß in der Fermenterlösung ein pH-Wert zwischen 6,2 und 7,2 eingestellt wird.

6. Verfahren nach Anspruch 1 oder einem der folgenden, dadurch gekennzeichnet, daß die Enzymabtrennung von Bakterienkulturen erfolgt, die sowohl in Gegenwart von Azid, insbesondere Natriumazid, als auch in Gegenwart von Natriumchlorid noch bei Temperaturen von 55° C zum Wachstum befähigt sind.

## Claims

1. A process for the removal of biotechnologically produced valuable materials from a cell suspension by crossflow microfiltration using porous polymeric tubes having micropores > 0.1 μm as membranes through which the fermenter solution flows at a rate of > 2 m/s, a pressure difference of less than 5 bar prevailing between the concentrate side and the permeate side, characterized in that, using polysulfone tubes having micropore diameters of 0.3 to 0.5 μm at a flow rate of the fermenter solution of 3 to 6 m/s parallel to the membrane surface and a pressure difference of 2 bar between the concentrate side and the permeate side, alkaline protease of relatively high molecular weight, more particularly enzyme > 20,000 dalton, is separated from a fermenter solution, the ratio of the average membrane pore diameter to the size of the microorganisms remaining in the concentrate being between 0.15 and 0.85.

2. A process as claimed in claim 1, characterized by an addition of 0.05 to 1% by weight solids having a particle size of ≦ 500 μm.

3. A process as claimed in claim 1 or 2, characterized by an addition of surfactants.

4. A process as claimed in any of the preceding claims, characterized in that the crossflow microfiltration is followed by ultrafiltration to concentrate the valuable materials.

5. A process as claimed in any of the preceding claims, characterized in that a pH value of 6.2 to 7.2 is adjusted in the fermenter solution.

6. A process as claimed in claim 1 or any of the following claims, characterized in that the enzyme is separated from bacterial cultures which are still capable of growth both in the presence of azide, particularly sodium azide, and in the presence of sodium chloride at temperatures of 55°C.

**Revendications**

1. Procédé pour la séparation de substances produites par biotechnologie á partir d'une suspension cellulaire par microfiltration de courant transversal en utilisant des tubes polymères poreux avec des micropores > 0,1 $\mu$m comme membrane, à travers lesquels la solution du fermentateur s écoule à une vitesse > 2 m/sec et où règne une différence de pression entre le côté du produit concentré et le côté du perméat de moins de 5 bars, caractérisé en ce qu'en utilisant des tubes de polysulfone comprenant des micropores de diamètre entre 0,3 et 0,5 $\mu$m à une vitesse de la solution du fermentateur entre 3 et 6 m/sec parallèlement à la surface de la membrane et une différence de pression entre le côté du produit concentré et le côté du perméat de 2 bars, une protéase alcaline de poids moléculaire plus élevé, en particulier une enzyme > 20 000 daltons est séparée d'une solution du fermentateur, le rapport du diamètre moyen des pores de la membrane par rapport à la grandeur des micro-organismes restant dans le concentré étant situé entre 0,15 et 0,85.

2. Procédé selon la revendication 1, caractérisé par une addition de 0,05 à 1 % en poids en matières solides de grandeur de particules $\leq$ 500 $\mu$m.

3. Procédé selon l'une des revendications 1 ou 2, caractérisé par une addition de tensio-actifs.

4. Procédé selon l'une des revendications précédentes, caractérisé en ce qu'à la suite de la microfiltration de courant transversal est intercalée une ultrafiltration pour l'enrichissement en matière.

5. Procédé selon l'une des revendications précédentes, caractérisé en ce qu'une valeur de pH entre 6,2 et 7,2 est ajustée dans la solution du fermentateur.

6. Procédé selon la revendication 1 ou l'une des revendications suivantes, caractérisé en ce que la séparation d'enzyme se fait par des cultures bactériennes qui sont susceptibles de croissance encore à des températures de 55°C aussi bien en présence d'acide, particulièrement d'azid de sodium, qu'également en présence de chlorure de sodium.

FIG.1

FIG.2